(19) Europäisches Patentamt
European Patent Office
Office européen des brevets

(11) Veröffentlichungsnummer: **0 363 796 B1**

(12) # EUROPÄISCHE PATENTSCHRIFT

(45) Veröffentlichungstag der Patentschrift: **11.08.93**

(21) Anmeldenummer: **89118382.4**

(22) Anmeldetag: **04.10.89**

(51) Int. Cl.5: **C07D 405/12**, C07D 407/12, C07D 409/12, C07D 413/12, C07D 417/12, A61K 31/35, A61K 31/40, A61K 31/41, A61K 31/445, A61K 31/44, A61K 31/47

(54) **Heterocyclische substituierte Alkoxycumarine, Verfahren zu ihrer Herstellung und diese enthaltende therapeutische Mittel.**

(30) Priorität: **13.10.88 DE 3834860**

(43) Veröffentlichungstag der Anmeldung:
**18.04.90 Patentblatt 90/16**

(45) Bekanntmachung des Hinweises auf die Patenterteilung:
**11.08.93 Patentblatt 93/32**

(84) Benannte Vertragsstaaten:
**AT BE CH DE FR GB IT LI NL**

(56) Entgegenhaltungen:
**DD-A- 258 228**
**FR-M- 6 457**

(73) Patentinhaber: **BASF Aktiengesellschaft**
**Carl-Bosch-Strasse 38**
**W-6700 Ludwigshafen(DE)**

(72) Erfinder: **Frickel, Fritz-Frieder, Dr.**
**II Craven Road**
**Mountain Lakes, N.J. 07046(US)**
Erfinder: **Kuekenhoehner, Thomas, Dr.**
**Seidelstrasse 2**
**W-6710 Frankenthal(DE)**
Erfinder: **Rendenbach-Mueller, Beatrice, Dr.**
**Kapellenstrasse 8**
**W-6701 Waldsee(DE)**
Erfinder: **Weifenbach, Harald, Dr.**
**Londoner Ring 71**
**W-6700 Ludwigshafen(DE)**
Erfinder: **Teschendorf, Hans-Juergen, Dr.**
**Georg-Nuss-Strasse 5**
**W-6724 Dudenhofen(DE)**

**EP 0 363 796 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 90, Nr. 19, 7. Mai 1979, Columbus, Ohio, USA HUSAIN, MOHAMED IMTIAZ et al.:"Search for potent anthelmin- tics. Part VIII. 4-Substituted-7-coumarinyloxyacetyl thiosemicarbazides and 7-coumarinyloxymethyl-1,2,4-tria- zoles and 1,3,4-oxadiazoles." Seite 605, Spalte 2, Zusammen-fassung-Nr. 152 084b

CHEMICAL ABSTRACTS, Band 77, Nr. 5, 31. Juli 1972, Columbus, Ohio, USA MURTHY, A. et al.: "Synthesis of 3-phenyl-5-aryloxymethyl- isoxazoles and their uv spectra and physiological activity." Seite 101, Spalte 1, Zusammenfassung-Nr. 29 615e

CHEMICAL ABSTRACTS, Band 99, Nr. 21, 21. November 1983, Columbus, Ohio, USA STACCHINO, C. et al.: "Spasmolytic activity of some 4-methylumbelliferone deriva- tives." Seite 54, Spalte 2, Zusammenfassung-Nr. 169 333r

CHEMICAL ABSTRACTS, Band 102, Nr. 9, 4. MUrz 1985, Columbus, Ohio, USA KULSHRESTHA, S.K. et al.: "Photo-induced reactions: Part IV - studies on photo Fries migration of some coumarins." Seite 534, Spalte 2, Zusammenfassung-Nr. 78 116m

## Beschreibung

Gegenstand der Erfindung sind neue heterocyclisch substituierte Alkoxycumarine der allgemeinen Formel I, deren Herstellung und therapeutische Mittel, die diese und eine bekannte Verbindung als Wirkstoff enthalten, insbesondere zur Behandlung zentralnervöser Erkrankungen.

Krishna Murthy et al., Indian J. Chem. 10, 38 bis 40 (1972) haben mit wenig bzw. gar keinem Erfolg 4-Methyl-7-(3-phenyl-isoxazol-5-yl-methoxy)cumarin auf fungizide und bakteriostatische Wirkung geprüft. Von dieser Verbindung abgesehen ist die hier beschriebene Verbindungsklasse neu, ebenso wie die therapeutische Verwendung der bekannten Verbindung.

Der Erfindung lag die Aufgabe zugrunde, neue therapeutische Mittel, insbesondere zur Behandlung zentralnervöser Erkrankungen zu entwicheln.

Die Lösung dieser Aufgabe besteht in den neuen Verbindungen der allgemeinen Formel I nach Anspruch 1, dem Herstellverfahren nach Anspruch 2 und den therapeutischen Mitteln nach den Ansprüchen 3 und 4.

In der allgemeinen Formel I

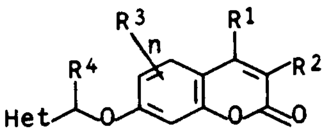 I,

bedeuten $R^1$ und $R^2$, die gleich oder verschieden sein können, Wasserstoff, Alkyl von $C_1$ bis $C_5$, wobei $R^1$ und $R^2$ eine gemeinsame Kette von 3-5 Kohlenstoffen bilden können, $CF_3$, Phenyl oder Halogen;
$R^3$ Alkyl von $C_1$ bis $C_5$, Halogen;
n eine ganze Zahl von 0 bis 3;
$R^4$ Wasserstoff oder Alkyl von $C_1$ bis $C_4$ und
Het einen der folgenden Heterocyclen:

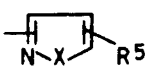 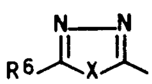 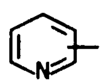 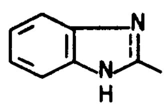 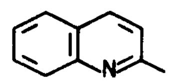 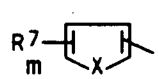

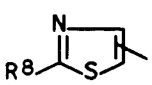 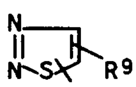 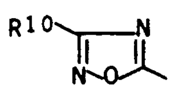 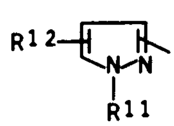 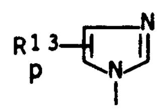 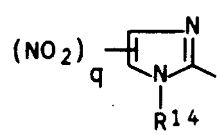

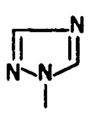 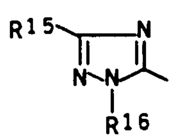 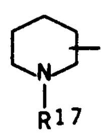 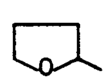 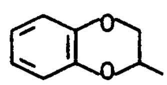 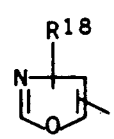 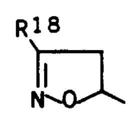

mit folgenden Bedeutungen für die benutzten Symbole:
X Sauerstoff oder Schwefel;
$R^5$
gegebenenfalls $C_1$-$C_4$-alkoxysubstituiertes $C_1$-$C_{10}$-Alkyl, 5-6-gliedriges Oxacycloalkyl, $C_3$-$C_7$-Cycloalkyl, Benzyl, einen gegebenenfalls durch Halogen oder $NO_2$ substituierten Phenylrest, eine Pyridinring, $C_1$-$C_5$-Alkoxycarbonyl, Perfluor$C_1$-$C_2$-alkyl;
$R^6$
$C_1$-$C_5$-Alkyl, $C_1$-$C_5$-Alkoxy;
$R^7$
$C_1$-$C_5$-Alkyl, einen gegebenenfalls halogensubstituierten Benzylrest, Halogen; m 0 bis 2;
$R^8$
$C_1$-$C_5$-Alkyl, $C_3$-$C_6$-Cycloalkyl, Benzyl, Phenyl;

$R^9$
Wasserstoff, $C_1$-$C_5$-Alkyl;
$R^{10}$
$C_1$-$C_5$-Alkyl, $C_1$-$C_6$-Cycloalkyl;
$R^{11}$
$C_1$-$C_5$-Alkyl;
$R^{12}$
Wasserstoff, $C_1$-$C_5$-Alkyl, $C_3$-$C_6$-Cycloalkyl;
$R^{13}$
$C_1$-$C_5$-Alkyl, Halogen, p = 0 bis 2;
$R^{14}$
$C_1$-$C_5$-Alkyl, q = 0 oder 1;
$R^{15}$, $R^{16}$
Wasserstoff, $C_1$-$C_5$-Alkyl, Benzyl;
$R^{17}$
Wasserstoff, $C_1$-$C_5$-Alkyl;
$R^{18}$
$C_1$-$C_5$ Alkyl
mit der Maßgabe, daß $R^5$ nicht Phenyl ist, wenn X Sauerstoff, $R^1$ Methyl und $R^2$ bis $R^4$ Wasserstoff darstellen.

Mit "Halogen" ist vzgw. Chlor oder Brom gemeint.

Die Verbindungen der allgemeinen Formel I lassen sich beispielsweise herstellen, indem man ein Hydroxycumarin der Formel II,

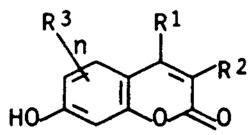

II,

in der $R^1$, $R^2$, $R^3$ und n die oben angegebenen Bedeutungen haben, in an sich bekannter Weise mit einer Verbindung der Formel III, umsetzt,

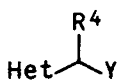

III,

in der $R^4$ und Het wie eingangs definiert sind und Y für eine nucleofuge Abgangsgruppe wie Chlor, Brom oder $R^{19}SO_2O$ steht. $R^{19}$ bedeutet hierin Niederalkyl oder gegebenenfalls durch $C_{1-3}$-Niederalkyl oder Halogen substituiertes Phenyl. Die Umsetzung kann, wie beispielsweise in Houben-Weyl, Georg Thieme-Verlag, Stuttgart, 1965, Bd. 6/3, S. 54 ff. beschrieben, durch Erhitzen der beiden Komponenten, vorzugsweise in Anwesenheit eines inerten Lösungsmittels wie Benzol, Toluol, Methylenchlorid, Aceton, einem niederen Alkohol, Dimethylformamid oder Wasser durchgeführt werden. Die Reaktionstemperaturen liegen zweckmäßigerweise zwischen der Raumtemperatur und dem Siedepunkt des verwendeten Lösungsmittels. Die freiwerdende Säure wird im allgemeinen durch Zusatz von Basen wie Alkali- oder Erdalkalihydroxiden oder carbonaten oder Aminen wie Pyridin oder Triethylamin abgefangen. Anstelle der Hydroxycumarine der Formel II können auch deren Alkalimetallsalze mit den Verbindungen der Formel III, vorzugsweise unter wasserfreien Bedingungen in aprotischen Lösungsmitteln wie Ether, Tetrahydrofuran, Dimethylformamid, Dimethoxyethan oder Dimethylsulfoxid, umgesetzt werden. Als Basen können in diesen Fällen Alkalimetallhydride oder -Alkoholate eingesetzt werden. Die Isolierung und Reinigung der Produkte erfolgt nach an sich bekannten Methoden, beispielsweise durch Umkristallisieren aus einem Lösungmittel, durch Säulenchromatographie oder gegebenenfalls Überführen in eine Säureadditionsverbindung, sofern Het basischen Charakter hat.

Die Hydroxycumarine II können nach bekannten Methoden, wie sie zum Beispiel in Elderfield R.C., Heterocyclic Compounds, John Wiley-Verlag, New York 1951, Bd. 2, S. 174f. beschrieben sind, hergestellt werden, beispielsweise durch Kondensation von Dihydroxybenzolen der Formel IV

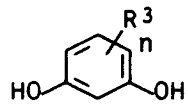

IV,

in der $R^3$ und n die oben angegebenen Bedeutungen haben, mit $\beta$-Ketocarbonsäureestern der Formel V

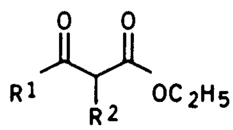

V,

in der $R^1$ und $R^2$ die oben angegebenen Bedeutungen besitzen, in Anwesenheit eines Kondensationsmittels wie Schwefelsäure, Phosphorpentoxid oder Aluminiumchlorid.

Die heterocyclischen Derivate der allgemeinen Formel III sind entweder bekannt und teilweise kommerziell erhältlich oder lassen sich nach allgemein bekannten chemischen Verfahren herstellen. Methoden zur Synthese von Thiophenderivaten finden sich beispielsweise in: Comprehensive Heterocyclic Chemistry, R. Katritzky und W. Rees, Vol. 4, S. 863 ff., Pergamon Press 1984; Furanderivate z.B. AU 579 693, DE-A-3 546 371 oder Advances in Heterocyclic Chemistry, Vol. 30, S. 167 ff., 1982; Thiazolderivate, Oxazolderivate, Isothiazolderivate, Thiadiazolderivate und Oxdiazolderivate z.B. Comprehensive Heterocyclic Chemistry, P. Katritzky und W. Rees, Vol. 6. S. 166, 177, 235, 386, 425 ff., Pergamon Press, 1984; Imidazolderivate z.B. Advances in Heterocyclic Chemistry, Vol. 27, S. 242 ff., 1980; Pyrazolderivate z.B. Heteroaromatic Nitrogen Compounds, The Azoles, S. 31 ff., Cambridge University Press, 1976; Triazolderivate z.B. Comprehensive Heterocyclic Chemistry, R. Katritzky und W. Rees, Vol. 5, S. 733 ff., Pergamon Press, 1984; Isoxazolderivate z.B. GB 1 560 711 und DE-A-2 754 832.

Falls die Verbindungen der allgemeinen Formel I basisch sind, können sie in die Säureadditionssalze einer physiologisch verträglichen Säure uberführt werden. Als übliche physiologisch verträgliche organische oder anorganische Säuren kommen beispielsweise Salzsäure, Bromwasserstoffsäure, Phosphorsäure, Schwefelsäure, Maleinsäure, Fumarsäure, Milchsäure, Weinsäure, Adipinsäure oder Benzoesäure in Betracht. Weitere können aus Fortschritte der Arzneimittelforschung, Bd. 10, S. 224 f., Birkhäuser Verlag, Basel und Stuttgart, 1966, entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Ethanol oder Propanol, oder einem Ether wie Diethyl- oder Methyl-t-butylether, erhalten. Zur besseren Kristallabscheidung können auch Mischungen der genannten Lösungsmittel verwendet werden. Darüber hinaus können gegebenenfalls pharmazeutisch vertretbare wäßrige Lösungen von Säureadditionsverbindungen der erfindungsgemäßen Verbindungen I durch Auflösen der freien Basen in einer wäßrigen Säurelösung hergestellt werden.

Die Verbindungen der allgemeinen Formel I haben Monoaminoxidase (MAO)-inhibierende Aktivität. Aufgrund dessen können sie zur Behandlung von zentralnervösen, insbesondere neurodegenerativen Erkrankungen und Parkinsonismus verwendet werden.

Die MAO hemmende Aktivität der erfindungsgemäßen Verbindungen kann unter Verwendung von Standardmethoden bestimmt werden. So erfolgte die Bestimmung der Monoaminoxidasen A und B in verdünntem Rattenhirnhomogenat, dem 1. unterschiedliche Konzentrationen der zu prüfenden Testsubstanzen und 2. $^{14}C$-Phenylethylamin bzw. $^{14}C$-Tryptamin in einer Konzentration von 0,4 $\mu$mol/l zugesetzt wurden. Dieser Ansatz wurde 20 min bei 37 °C inkubiert.

Die Reaktion wurde dann durch 0,1 normale HCl gestoppt und die Reaktionsprodukte nach Extraktion im Toluol-Szintillator (PPO + POPOP in Toluol) bestimmt. Der Blindwert wurde in analogen Ansätzen mit einer Inkubationszeit von t = 0 min bestimmt.

Aus den bei den verschiedenen Inhibitor-Konzentrationen gegen die Kontrolle ermittelten Hemmwerten wurde durch lineare Regression nach logit-log-Transformation die mittlere Hemmkonzentration (IC50) berechnet.

Die so ermittelte Aktivität einiger erfindungsgemäßer Verbindungen ist aus der folgenden Tabelle ersichtlich:

| Bsp. | IC50 [µmol/l] | | MAO A / MAO B |
|---|---|---|---|
| | MAO A | MAO B | |
| 1 | 5,6 | 0,015 | 370 |
| 2 | 3,6 | 0,003 | 1200 |
| 3 | 1,5 | 0,0021 | 710 |
| 4 | ~ 10 | 0,0017 | ~ 5900 |
| 6 | ~ 10 | 0,0046 | ~ 2200 |
| 7 | ~ 10 | 0,011 | ~ 900 |
| 10 | > 10 | 0,0022 | > 4500 |
| 13 | > 10 | 0,015 | > 667 |
| 21 | ~ 10 | 0,0077 | ~ 1300 |
| 22 | > 10 | 0,0016 | > 6250 |
| 24 | ~ 10 | 0,003 | ~ 3300 |
| 25 | > 10 | 0,017 | > 590 |
| 26 | > 10 | 0,0013 | > 7700 |
| 29 | > 10 | 0,0022 | > 4500 |
| 30 | > 10 | 0,0057 | > 1700 |
| 32 | > 10 | 0,0071 | > 1400 |
| 33 | ~ 6 | 0,00073 | ~ 8200 |
| 34 | ~ 10 | 0,0022 | ~ 4500 |
| 35 | > 10 | 0,00092 | >11000 |
| 42 | 0,35 | 0,00051 | 680 |
| 44 | ~ 7 | 0,0012 | ~ 5800 |
| 45 | 0,23 | 0,00051 | 450 |
| 50 | ~ 4 | 0,0043 | ~ 930 |
| 51 | > 10 | 0,0008 | >12500 |
| 54 | > 10 | 0,0015 | > 6700 |
| 55 | > 10 | 0,01 | > 1000 |
| 58 | ~ 7 | 0,00084 | ~ 8300 |
| 61 | > 10 | 0,0019 | > 1100 |
| 62 | > 10 | 0,0075 | > 1300 |
| 67 | > 10 | 0,0011 | > 9100 |
| 68 | > 10 | 0,00077 | >13000 |
| 70 | > 10 | 0,013 | > 770 |
| 72 | ~ 10 | 0,0049 | ~ 2000 |
| 73 | > 10 | 0,014 | > 700 |
| 74 | 3,2 | 0,0055 | 580 |
| 75 | ~ 10 | 0,0014 | ~ 7000 |
| 78 | > 10 | 0,0013 | > 7700 |
| 79 | 2,9 | 0,0012 | 2400 |
| 80 | ~ 10 | 0,0014 | ~ 7100 |

| Bsp. | IC50 [µmol/l] | | MAO A / MAO B |
|---|---|---|---|
| | MAO A | MAO B | |
| 84 | > 10 | 0,0091 | > 1100 |
| 85 | > 10 | 0,0069 | > 1700 |
| 87 | > 10 | 0,014 | > 700 |
| 92 | > 10 | ~ 0,01 | > 1000 |
| 94 | ~ 10 | 0,0012 | ~ 8300 |
| 96 | ~ 20 | 0,00061 | ~32000 |
| 97 | ~ 5 | 0,0036 | ~ 1400 |
| 98 | > 10 | 0,00092 | >11000 |
| 99 | > 10 | 0,0082 | > 1200 |
| 100 | > 10 | 0,00074 | >13000 |
| 101 | > 10 | 0,001 | >10000 |
| 103 | > 10 | 0,0018 | > 5500 |
| 108 | ~ 10 | 0,0009 | ~11000 |
| 109 | > 10 | 0,00088 | >11000 |
| Deprenyl | 2,0 | 0,0078 | 256 |
| Ro 19-6327 | > 10 | 0,022 | > 450 |

Die erfindungsgemäßen Verbindungen können in üblicher Weise oral oder parenteral (subkutan, intravenös, intramuskulär, intraperitoneal) verabfolgt werden.

Die Dosierung hängt vom Alter, Zustand und Gewicht des Patienten sowie von der Applikationsart ab. In der Regel beträgt die tägliche Wirkstoffdosis zwischen etwa 10 und 500 mg pro Patient und Tag bei oraler und zwischen etwa 1 und 50 mg pro Patient und Tag bei parenteraler Gabe.

Die neuen Verbindungen können in den gebräuchlichen galenischen Applikationsformen fest oder flüssig angewendet werden, z. B. als Tabletten, Filmtabletten, Kapseln, Pulver, Granulate, Dragees, Suppositorien, Lösungen oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sukker et al.: Pharmazeutische Technologie, Thieme-Verlag, Stuttgart, 1978). Die so erhaltenen Applikationsformen enthalten den Wirkstoff normalerweise in einer Konzentration von 1 bis 99 Gew.-%.

Beispiel 1

3,4-Dimethyl-7-(2-methyl-1,3,4-oxdiazol-5-yl)-methoxycumarin

5,0 g 7-Hydroxy-3,4-dimethylcumarin in 20 ml DMF wurden bei Raumtemperatur zu einer Suspension von 0, 9 g NaH (80 %) in 50 ml DMF zugetropft. Nach 45 min wurde mit 3,5 g 2-Chlormethyl-5-methyl-1,3,4-oxdiazol, gelöst in 20 ml DMF, versetzt und über Nacht bei RT gerührt. Die Reaktionslösung wurde mit Eiswasser hydrolysiert, der ausgefallene Feststoff abgesaugt und aus Methanol umkristallisiert.
Ausbeute: 4,28 g (57 %), Fp. 159 °C
$C_{15}H_{14}N_2O_4$ (286)
Ber. 62,93 C 4,93 H 9,78 N 22,35 O
Gef. 63,0 C 4,9 H 9,7 N 22,2 O

Beispiel 2

3,4-Dimethyl-7-(2-methyl-1,3,4-thiadiazol-5-yl)-methoxycumarin

Ein Gemisch aus 5,0 g 7-Hydroxy-3,4-dimethylcumarin, 3,9 g 2-Chlormethyl-5-methyl-1,3,4-thiadiazol, 3,5 g $K_2CO_3$ und 100 ml Aceton wurde 20 h am Rückfluß gekocht, eingeengt und der Rückstand in $H_2O$/Methylenchlorid verteilt. Nach Abtrennen der ungelösten Bestandteile wurde die organische Phase 1 x mit 1n NaOH und anschließend mit $H_2O$ gewaschen, über $Na_2SO_4$ getrocknet, eingeengt und der Rückstand aus Methanol umkristallisiert und getrocknet.
Ausbeute: 5,8 (74 %), Fp. 150 ° C
$C_{15}H_{14}N_2O_3S$ (302)
Ber. 59,59 C 4,67 H 9,27 N 15,87 O 10,60 S
Gef. 59,4 C 4,5 H 9,4 N 16,0 O 10,6 S
Analog Beispiel 2 wurde hergestellt:

Beispiel 3

3,4-Dimethyl-7-(3-methylisoxazol-5-yl)-methoxycumarin

Ausbeute: 43 %; Fp. 148-150 ° C (Ethanol)
$C_{16}H_{15}NO_4$ (285)
Ber. 67,36 C 5,30 H 4,91 N 22,43 O
Gef. 67,2 C 5,4 H 5,1 N 22,3 O
Anlog Beispiel 1 wurden hergstellt:

Beispiel 4

3,4-Dimethyl-7-(3-n-propylisoxazol-5-yl)-methoxycumarin

Ausbeute: 50 %; Fp. 94 ° C (Methanol)
$C_{18}H_{19}NO_4$ (313)
Ber. 68,99 C 6,11 H 4,47 N 20,42 O
Gef. 68,7 C 6,2 H 4,4 N 20,2 O

Beispiel 5

3,4-Dimethyl-7-(4-pyridinyl)-methoxycumarin

Ausbeute: 52 %; Fp. 171 ° C (Methanol)
$C_{17}H_{15}NO_3$ (281)
Ber. 72,58 C 5,37 H 4,98 N 17,06 O
Gef. 72,3 C 5,5 H 4,8 N 16,9 O

Beispiel 6

7- (3-Methoxymethylisoxazol-5-yl)-methoxy-3,4-dimethylcumarin

Ausbeute 70 %; Fp. 133 ° C (Methanol)
$C_{17}H_{17}NO_5$ (315)
Ber. 64,75 C 5,43 H 4,44 N 25,37 O
Gef. 64,7 C 5,5 H 4,2 N 25,0 O

Beispiel 7

7-(2-Ethyl-1,3,4-oxdiazol-5-yl)-methoxy-3,4-dimethylcumarin

Ausbeute: 54 %; Fp. 118 ° C (Methanol)
$C_{16}H_{16}N_2O_4$ (300)

Ber. 63,99 C 5,37 H 9,33 N 21,31 O
Gef. 63,7 C 5,8 H 9,3 N 21,3 O

Beispiel 8

3,4-Dimethyl-7-(2-pyridinyl)-methoxycumarin

Ausbeute: 76 %; Fp. 156 °C (Methanol)
$C_{17}H_{15}NO_3$ (281)
Ber. 72,58 C 5,37 H 4,98 N 17,06 O
Gef. 72,4 C 5,5 H 5,0 N 16,9 O

Beispiel 9

3,4-Dimethyl-7-(3-pyridinyl)-methoxycumarin

Ausbeute: 64 %; Fp. 154 °C (Methanol)
$C_{17}H_{15}NO_3$ (281)
Ber. 72,58 C 5,37 H 4,98 N 17,06 O
Gef. 72,4 C 5,6 H 4,9 N 16,9 O

Beispiel 10

3,6-Dichlor-4-methyl-7-(2-cyclopropylthiazol-4-yl)-methoxycumarin

Durchführung und Aufarbeitung erfolgten analog Beispiel 2. Zur Reaktionsmischung wurden zusätzlich 0,1 % 18-Krone-6 zugefügt.
Ausbeute: 26 %; Fp. 182 - 184 °C (Methanol)
$C_{17}H_{13}Cl_2NO_3S$ (382)
Ber. 53,41 C 3,43 H 18,55 Cl 3,66 N 12,56 O 8,39 S
Gef. 53,0 C 3,5 H 19,0 Cl 3,6 N 12,4 O 8,1 S

Beispiel 11

7-(2-Chlorthiophen-5-yl)-methoxy-3,4-dimethylcumarin

Ausbeute: 69 %; Fp. 152-154 °C (Methanol)
$C_{16}H_{13}ClO_3S$ (321)
Ber. 59,91 C 4,08 H 11,05 N 14,96 O 10,00 S
Gef. 59,4 C 4,2 H 11,0 N 14,8 O 10,0 S

Beispiel 12

7-(2-Methyl-1,3,4-thiadiazol-5-yl)-methoxycumarin

Ausbeute: 64 %; Fp. 155 °C (Methanol)
$C_{13}H_{10}N_2O_3S$ (274)
Ber. 56,92 C 3,67 H 10,21 N 17,50 O 11,69 S
Gef. 56,6 C 3,6 H 10,3 N 17,4 O 11,6 S

Beispiel 13

3-Methyl-7-(2-methyl-1,3,4-thiadiazol-5-yl)-methoxycumarin

Ausbeute: 59 %; Fp. 177 °C (Methanol)
$C_{14}H_{12}N_2O_3S$ (288)
Ber. 58,32 C 4,19 H 9,72 N 16,65 O 11,12 S
Gef. 58,1 C 4,3 H 9,6 N 16,7 O 11,3 S

Beispiel 14

4-Methyl-7-(2-methyl-1,3,4-thiadiazol-5-yl)-methoxycumarin

Ausbeute: 56 %; Fp. 143-144 ° C (Methanol)
$C_{14}H_{12}N_2O_3S$ (288)
Ber. 58,32 C 4,20 H 9,72 N 16,65 O 11,12 S
Gef. 58,4 C 4,4 H 9,6 N 16,5 O 10,9 S

Beispiel 15

7-(Benzimidazol-2-yl)-methoxy-3,4-dimethylcumarin

Ausbeute: 20 %; Fp. 271-274 ° C (Methanol)
$C_{19}H_{16}N_2O_3$ (320)
Ber. 71,24 C 5,03 H 8,74 N 14,98 O
Gef. 70,8 C 5,3 H 8,6 N 15,2 O

Beispiel 16

7-(Chinolin-2-yl)-methoxy-3,4-dimethylcumarin

Ausbeute: 66 %; Fp. 189-190 ° C (Methanol)
$C_{21}H_{17}NO_3$ (331)
Ber. 76,12 C 5,17 H 4,23 N 14,48 O
Gef. 76,1 C 5,2 H 4,2 N 14,3 O

Beispiel 17

3,4-Dimethyl-7-(1-methylpiperidin-3-yl)-methoxycumarin

Die Reaktionsmischung rührte 12 h bei 120 ° C. Ansatz und Aufarbeitung erfolgten wie unter Beispiel 1 beschrieben. Ausbeute: 55 %; Fp. 126-128 ° C (Methanol)
$C_{18}H_{23}NO_3$ (301)
Ber. 71,73 C 7,69 H 4,65 N 15,93 O
Gef. 71,8 C 7,8 H 4,5 N 15,8 O

Beispiel 18

7-(Tetrahydrofuran-2-yl)-methoxy-3,4-dimethylcumarin

Die Reaktionsmischung rührte 12 h bei 120 ° C. Ansatz und Aufarbeitung erfolgten wie unter Beispiel 1 beschrieben. Ausbeute: 55 %; Fp. 151-153 ° C (Methanol)
$C_{16}H_{18}O_4$ (274)
Ber. 70,06 C 6,61 H 23,33 O
Gef. 70.1 C 6,7 H 23,2 O

Beispiel 19

7-[3-(3,4-Dichlorphenyl)-isoxazol-5-yl]-methoxy-3,4-dimethylcumarin

Ausbeute: 56 %; Fp. 225 ° C
$C_{21}H_{15}Cl_2NO_4$ (416)
Ber. 60,59 C 3,63 H 17,03 Cl 3,36 N 15,37 O
Gef. 60,2 C 3,8 H 17,2 Cl 3,3 N 15,4 O

Beispiel 20

3,4-Dimethyl-7-[3-(3-Nitrophenyl)-isoxazol-5-yl]-methoxycumarin

Ausbeute: 20 %; Fp. 212-219 °C (Zers.)
$C_{21}H_{16}N_2O_2$ (392)
Ber. 64,28 C 4,11 H 7,14 N 24,47 O
Gef. 63,8 C 4,5 H 6,9 N 24,0 O

Beispiel 21

7-(Benzodioxan-2-yl)-methoxy-3,4-dimethylcumarin

Die Reaktionsmischung wurde 12 h bei 120 °C gerührt. Ansatz und Aufarbeitung erfolgten wie unter Beispiel 1 beschrieben.
Ausbeute: 53 %; Fp. 145 °C (Ethanol)
$C_{20}H_{18}O_5$ (338)
Ber. 71,0 C 5,36 H 23,64 O
Gef. 70,8 C 5,4 H 23,4 O

Beispiel 22

7-(2-Benzylfuran-4-yl)-methoxy-3,4-dimethylcumarin

Ausbeute: 68 %; Fp. 113 °C (Methanol)
$C_{23}H_{20}O_4$ (360)
Ber. 76,65 C 5,59 H 17,76 O
Gef. 76,4 C 5,6 H 17,7 O

Beispiel 23

7-(2-Methyl-1,3,4-thiadiazol-5-yl)-methoxy-4-phenylcumarin

Die Reaktionsmischung rührte 3 h bei 100 °C und über Nacht bei RT. Ansatz und Durchführung erfolgten wie unter Beispiel 1 beschrieben.
Ausbeute: 80 %; Fp. 166-167 °C (Methanol)
$C_{19}H_{14}N_2O_3S$ (350)
Ber. 65,13 C 4,03 H 7,99 N 13,70 O 9,15 S
Gef. 65,3 C 4,1 H 8,0 N 13,9 O 9,1 S

Beispiel 24

3,4-Dimethyl-7-(2-methylthiazol-4-yl)-methoxycumarin

Ausbeute: 69 %; Fp. 149-150 °C (Methanol)
$C_{16}H_{15}NO_3S$ (301)
Ber. 63,77 C 5,02 H 4,65 N 15,93 O 10,64 S
Gef. 63,5 C 5,1 H 4,6 N 16,1 O 10,4 S

Beispiel 25

7-[2-(3-Chlorbenzyl)-furan-4-yl]-methoxy-3,4-dimethylcumarin

Ausbeute: 45 %; Fp. 115 °C (Methanol)
$C_{23}H_{19}ClO_4$ (395)
Ber. 69,96 C 4,85 H 8,98 Cl 16,21 O
Gef. 69,9 C 5,1 H 8,8 Cl 16,5 O

Beispiel 26

7-(3-Isopropyl-1,2,4-oxdiazol-5-yl)-methoxy-3,4-dimethylcumarin

Ausbeute: 82 %; Fp. 142 °C (Methanol)
$C_{17}H_{18}N_2O_4$ (314)
Ber. 64,96 C 5,77 H 8,91 N 20,36 O
Gef. 64,8 C 5,9 H 8,9 N 20,4 O

Beispiel 27

3,4-Dimethyl-7-[3-(tetrahydropyran-4-yl)-isoxazol-5-yl]-methoxycumarin

Ausbeute: 77 %; Fp. 151 °C (Methanol)
$C_{20}H_{21}NO_5$ (355)
Ber. 67,59 C 5,96 H 3,94 N 22,51 O
Gef. 67,5 C 5,9 H 4,0 N 22,5 O

Beispiel 28

7-(4,5-Dihydro-3-propylisoxazol-5-yl)-methoxy-3,4-dimethylcumarin

Die Reaktionsmischung wurde 8 h bei 100 °C gerührt. Ansatz und Aufarbeitung erfolgten wie unter Beispiel 1 beschrieben.
Ausbeute: 60 %; Fp. 147 °C (Methanol)
$C_{18}H_{21}NO_4$ (315)
Ber. 68,55 C 6,71 H 4,44 N 20,29 O
Gef. 68,3 C 6,7 H 4,3 N 20,0 O

Beispiel 29

7-(5-Isopropyl-1-methylpyrazol-3-yl)-methoxy-3,4-dimethylcumarin

Ausbeute: 43 %; Fp. 113 °C (Methanol)
$C_{19}H_{22}N_2O_3$ (326)
Ber. 69,92 C 6,79 H 8,58 N 14,71 O
Gef. 69,5 C 6,9 H 8,4 N 14,6 O

Beispiel 30

3-Chlor-4-methyl-7-(2-methyl-1,3,4-thiadiazol-5-yl)-methoxycumarin

Ausbeute: 62 %; Fp. 176-177 °C (Methanol)
$C_{14}H_{11}ClN_2O_3S$ (323)
Ber. 52,10 C 3,44 H 10,98 Cl 8,68 N 14,87 O 9,93 S Gef. 52,2 C 3,6 H 10,8 Cl 8,7 N 15,2 O 9,2 S

Beispiel 31

3-Ethyl-4-methyl-7-(2-methyl-1,3,4-thiadiazol-5-yl)-methoxycumarin

Ausbeute: 78 %; Fp. 167 °C (Methanol)
$C_{16}H_{16}N_2O_3S$ (316)
Ber. 60,74 C 5,10 H 8,85 N 15,17 O 10,13 S
Gef. 60,6 C 5,2 H 9,0 N 15,1 O 10,1 S

Beispiel 32

3,4-Tetramethylen-7-(2-methyl-1,3,4-thiadiazol-5-yl)-methoxycumarin

Ausbeute: 81 %; Fp. 172-174 °C (Methanol)
$C_{17}H_{16}N_2O_3S$ (328)
Ber. 62,18 C 4,91 H 8,53 N 14,62 O 9,76 S
Gef. 61,8 C 5,1 H 8,7 N 14,8 O 9,7 S

Beispiel 33

7-(3-Isopropylisoxazol-5-yl)-methoxy-3,4-dimethylcumarin

Ausbeute: 62 %; Fp. 105 °C (Methanol)
$C_{18}H_{19}NO_4$ (313)
Ber. 69,00 C 6,11 H 4,47 N 20,42 O
Gef. 68,8 C 6,3 H 4,4 N 20,4 O

Beispiel 34

7-(3-Isobutylisoxazol-5-yl)-methoxy-3,4-dimethylcumarin

Ausbeute: 79 %; Fp. 86 °C (Methanol)
$C_{19}H_{21}NO_4$ (327)
Ber. 69,71 C 6,47 H 4,28 N 19,55 O
Gef. 69,7 C 6,7 H 4,3 N 19,5 O

Beispiel 35

7-(3-t-Butylisoxazol-5-yl)-methoxy-3,4-dimethylcumarin

Ausbeute: 58 %; Fp. 121 °C (Methanol)
$C_{19}H_{21}NO_4$ (327)
Ber. 69,71 C 6,47 H 4,28 N 19,55 O
Gef. 69,7 C 6,7 H 4,3 N 19,4 O

Beispiel 36

7-(3-Ethoxycarbonylisoxazol-5-yl)-methoxy-3,4-dimethylcumarin

Ausbeute: 71 %; Fp. 202 °C
(Methylenchlorid/Essigsäureethylester)
$C_{18}H_{17}NO_6$ (343)
Ber. 62,97 C 4,99 H 4,08 N 27,96 O
Gef. 62,7 C 5,1 H 4,0 N 27,8 O

Beispiel 37

7-(4,5-Dichlorimidazol-1-yl)-methoxy-3,4-dimethylcumarin

Ausbeute: 84 %; Fp. 235 °
(Methylenchlorid/Essigsäureethylester)
$C_{15}H_{12}Cl_2N_2O_3$ (339)
Ber. 53,12 C 3,57 H 20,91 Cl 8,26 N 14,15 O
Gef. 52,2 C 3,6 H 20,6 Cl 8,0 N 14,2 O

Beispiel 38

3,4-Dimethyl-7-(1,2,4-triazol-1-yl)-methoxycumarin

Ausbeute: 73 %; Fp. 223-225 °C (Methanol)
$C_{14}H_{13}N_2O_3$ (271)
Ber. 61,99 C 4,83 H 15,49 N 17,69 O
Gef. 61,8 C 5,0 H 15,6 N 17,5 O

Beispiel 39

3,4-Dimethyl-7-(-4,5-dimethylimidazol-1-yl)-methoxycumarin

Ausbeute: 37 %; Fp. 196-198 °C (Essigsäureethylester)
$C_{17}H_{18}N_2O_3$ (298)
Ber. 68,44 C 6,08 H 9,39 N 16,09 O
Gef. 68,1 C 6,2 H 9,4 N 16,0 O

Beispiel 40

3,4-Dimethyl-7-(thiophen-2-yl)-methoxycumarin

Ausbeute: 43 %; Fp. 169-172 °C (Methanol)
$C_{16}H_{14}O_3S$ (286)
Ber. 67,11 C 4,93 H 16,76 O 11,20 S
Gef. 67,0 C 5,0 H 16,8 O 11,2 S

Beispiel 41

3,4-Dimethyl-7-(thiophen-3-yl)-methoxycumarin

Ausbeute: 41 %; Fp. 158-160 °C (Methanol)
$C_{16}H_{14}O_3S$ (286)
Ber. 67,11 C 4,93 H 16,76 O 11,20 S
Gef. 66,9 C 5,1 H 16,8 0 11,1 S

Beispiel 42

3,4-Dimethyl-7-(2-methylthiophen-5-yl)-methoxycumarin

Ausbeute: 66 %; Fp. 123-126 °C (Methanol)
$C_{17}H_{16}O_3S$ (300)
Ber. 67,98 C 5,37 H 15,98 O 10,67 S
Gef. 67,8 C 5,5 H 15,9 O 10,7 S

Beispiel 43

7-(2-Chlorthiophen-4-yl)-methoxy-3,4-dimethylcumarin

Ausbeute: 53 %; Fp. 137-139 °C (Methanol)
$C_{16}H_{13}ClO_3S$ (321)
Ber. 59,91 C 4,08 H 11,05 Cl 14,96 O 10,0 S
Gef. 59,6 C 4,1 H 11,0 Cl 15,1 O 9,9 S

Beispiel 44

3,4-Dimethyl-7-(3-cyclopropyl-1,2,4-oxdiazol-5-yl)-methoxycumarin

Ausbeute: 41 %; Fp. 128-131 ° C (Methanol)
$C_{17}H_{16}N_2O_4$ (312)
Ber. 65,38 C 5,16 H 8,97 N 20,49 O
Gef. 65,1 C 5,2 H 9,0 N 20,6 O

Beispiel 45

3,4-Dimethyl-7-(3-methylthiophen-2-yl)-methoxycumarin

Ausbeute: 52 %; Fp. 147 ° C (Methanol)
$C_{17}H_{16}O_3S$ (300)
Ber. 67,98 C 5,37 H 15,98 O 10,67 S
Gef. 68,0 C 5,4 H 15,9 O 10,7 S

Beispiel 46

7-(2-Bromthiophen-5-yl)-methoxy-3,4-dimethylcumarin

Ausbeute: 65 %; Fp. 140-143 ° C (Essigsäureethylester)
$C_{16}H_{13}BrO_3S$ (365)
Ber. 52,62 C 3,59 H 21,88 Br 13,14 O 8,78 S
Gef. 52,7 C 3,9 H 21,7 Br 13,3 O 8,8 S

Beispiel 47

7-(2-Bromthiophen-4yl)-methoxy-3,4-dimethylcumarin

Ausbeute: 67 %; Fp. 104 ° C (Ethanol)
$C_{16}H_{13}BrO_3S$ (365)
Ber. 52,62 C 3,59 H 21,88 Br 13,14 O 8,78 S
Gef. 52,7 C 3,7 H 21,4 Br 13,4 O 8,8 S

Beispiel 48

7-(4-Bromthiophen-2-yl)-methoxy-3,4-dimethylcumarin

Ausbeute: 53 %; Fp. 131-134 ° C (Methanol)
$C_{16}H_{13}BrO_3S$ (365)
Ber. 52,62 C 3,59 H 21,88 Br 13,14 O 8,78 S
Gef. 52,3 C 3,7 H 21,5 Br 13,3 O 8,7 S

Beispiel 49

7-(2,3-Dibromthiophen-4-yl)-methoxy-3,4-dimethylcumarin

Ausbeute: 57 %; Fp. 194-196 ° C (Methanol)
$C_{16}H_{12}Br_2O_3S$ (444)
Ber. 43,27 C 2,72 H 35,98 Br 10,81 O 7,22 S
Gef. 43,2 C 2,7 H 35,9 Br 11,1 O 7,1 S

Beispiel 50

3,4-Dimethyl-7-(3-methyl-1,2,4-oxdiazol-5-yl)-methoxycumarin

Ausbeute: 63 %; Fp. 172-174 °C (Methanol)
$C_{15}H_{14}N_2O_4$ (286)
Ber. 62,93 C 4,89 H 9,79 N 22,37 O
Gef. 62,6 C 5,0 H 9,8 N 22,9 O

Beispiel 51

7-(3-Cyclopropylisoxazol-5-yl)-methoxy-3,4-dimethylcumarin

Ausbeute: 36 %; Fp. 158-160 °C (Methanol)
$C_{18}H_{17}NO_4$ (311)
Ber. 69,44 C 5,50 H 4,50 N 20,56 O
Gef. 69,0 C 5,6 H 4,5 N 21,2 O

Beispiel 52

3,4-Dimethyl-7-(3-methylisothiazol-4-yl)-methoxycumarin

Ausbeute: 28 %; Fp. 163-165 °C (Methanol)
$C_{16}H_{15}NO_3S$ (301)
Ber. 63,77 C 5,02 H 4,65 N 15,93 O 10,64 S
Gef. 63,9 C 5,1 H 4,7 N 15,5 O 10,5 S

Beispiel 53

3,4-Dimethyl-7-(1,2,3-thiadiazol-4-yl)-methoxycumarin

Ausbeute: 39 %; Fp. 171-173 °C (Methanol)
$C_{14}H_{12}N_2O_3S$ (288)
Ber. 56,51 C 4,38 H 10,14 N 17,37 O 11,6 S
Gef. 56,9 C 4,1 H 9,6 N 17,7 O 11,8 S

Beispiel 54

3-Chlor-7-(5-isopropyl-1-methylpyrazol-3-yl)-methoxy-4-methylcumarin

Die Durchführung der Reaktion erfolgte wie unter Beispiel 1 beschrieben. Nach der Hydrolyse wurde mit Methylenchlorid extrahiert, die organische Phase mit $H_2O$ gewaschen, über $Na_2SO_4$ getrocknet, eingeengt und der Rückstand aus Methanol umkristallisiert.
Ausbeute: 33 %; Fp. 162-165 °C
$C_{18}H_{19}ClN_2O_3$ (346,5)
Ber. 62,34 C 5,52 H 8,08 N 13,84 O 10,22 S
Gef. 62,3 C 5,8 H 8,1 N 13,7 O 9,7 S

Beispiel 55

7-(5-Isopropyl-1-methylpyrazol-3-yl)-methoxy-3,4-tetramethylencumarin

Ausbeute: 29 %; Fp. 138-140 °C (Methanol)
$C_{21}H_{24}N_2O_3$ (352)
Ber. 71,57 C 6,86 H 7,95 N 13,62 O
Gef. 71,0 C 6,9 H 8,0 N 14,1 O

Beispiel 56

4-Ethyl-3-methyl-7-(2-methyl-1,3,4-thiadiazol-5-yl)-methoxycumarin

Ausbeute: 24 %; Fp. 135-138 °C (Methanol)
$C_{16}H_{16}N_2O_3S$ (316)
Ber. 60.74 C 5,10 H 8,85 N 15,17 O 10,13 S
Gef. 61,0 C 5,5 H 8,5 N 15,0 O 9,8 S

Beispiel 57

3,4-Dimethyl-7-(4-methyl-1,2,3-thiadiazol-5-yl)-methoxy cumarin

Ausbeute: 29 %; Fp. 204-206 °C (Methanol)
$C_{15}H_{14}N_2O_3S$ (302)
Ber. 59,60 C 4,63 H 9,27 N 15,89 O 10,59 S
Gef. 59,3 C 4,8 H 9,3 N 15,9 O 10,7 S

Beispiel 58

7-(5-Cyclopropylisoxazol-3-yl)-methoxy-3,4-dimethylcumarin

Ausbeute: 67 %; Fp. 107-109 °C (Methanol)
$C_{18}H_{17}NO_4$ (311)
Ber. 69,44 C 5,50 H 4,50 N 20,56 O
Gef. 69,1 C 5,8 H 4,3 N 20,4 O

Beispiel 59

7-(3-Trifluormethylisoxazol-5-yl)-methoxy-3,4-dimethylcumarin

Ausbeute: 47 %; Fp. 180-182 °C (Methanol)
$C_{16}H_{12}F_3NO_4$ (339)
Ber. 56,64 C 3,57 H 16,80 F 4,13 N 18,86 O
Gef. 56,6 C 3,7 H 16,8 F 4,0 N 18,9 O

Beispiel 60

3,4-Dimethy-7-(1-methylimidazol-2-yl)-methoxycumarin

Durchführung und Aufarbeitung erfolgten wie unter Beispiel 54 beschrieben.
Ausbeute: 28 %, Fp. 182-185 °C
$C_{16}H_{16}N_2O_3$ (284)
Ber. 67,59 C 5,67 H 9,85 N 16,88 O
Gef. 67,2 C 6,0 H 9,7 N 16,5 O

Beispiel 61

7-(5-Cyclopropyl-1-methylpyrazol-3-yl)-methoxy-3,4-dimethylcumarin

Ausbeute: 51 %; Fp. 124-127 °C (Methanol)
$C_{19}H_{20}N_2O_3$ (324)
Ber. 70,35 C 6,21 H 8,64 N 14,80 O
Gef. 70,1 C 6,4 H 8,7 N 14,6 O

Beispiel 62

7-(2-Ethoxy-1,3,4-thiadiazol-5-yl)-methoxy-3,4-dimethylcumarin

Ausbeute: 37 %; Fp. 174-176 °C (Methanol)
$C_{16}H_{16}N_2O_4S$ (332)
Ber. 57,82 C 4,85 H 8,43 N 19,25 O 9,65 S
Gef. 58,3 C 5,3 H 8,1 N 18,9 O 9,4 S

Beispiel 63

3,4-Dimethyl-7-(2-phenylthiazol-4-yl)-methoxycumarin

Durchführung und Aufarbeitung erfolgten wie unter Beispiel 54 beschrieben.
Ausbeute: 39 %; Fp. 152-154 °C (Methanol)
$C_{21}H_{17}NO_3S$ (363)
Ber. 69,40 C 4,71 H 3,85 N 13,21 O 8,82 S
Gef. 69,1 C 4,9 H 3,7 N 13,1 O 9,0 S

Beispiel 64

4-Ethyl-7-(5-isopropyl-1-methylpyrazol-3-yl)-methoxy-3-methylcumarin

Durchführung und Aufarbeitung erfolgten wie unter Beispiel 54 beschrieben.
Ausbeute: 33 %; Fp. 101-103 °C
$C_{20}H_{24}N_2O_3$ (340)
Ber. 70,57 C 7,11 H 8,23 N 14,10 O
Gef. 70,2 C 7,5 H 8,3 N 13,7 O

Beispiel 65

6-Chlor-3,4-dimethyl-7-(2-methyl-1,3,4-thiadiazol-5-yl)-methoxycumarin
Ausbeute: 37 %; Fp. 209-210 °C (Methanol)
$C_{15}H_{13}ClN_2O_3S$ (336,5)
Ber. 53,49 C 3,89 H 10,53 Cl 8,32 N 14,25 O 9,52 S Gef. 53,3 C 3,9 H 10,5 Cl 8,2 N 14,1 O 9,6 S

Beispiel 66

3,4-Dimethyl-7-(4-methylisothiazol-5-yl)-methoxycumarin

Ausbeute: 25 %, Fp. 180 °C (Methanol)
$C_{16}H_{15}NO_3S$ (301)
Ber. 63,56 C 5,33 H 4,63 N 15,87 O 10,6 S
Gef. 63,5 C 5,3 H 4,8 N 15,3 O 10,9 S

Beispiel 67

6-Brom-3,4-dimethyl-7-(2-methyl-1,3,4-thiadiazol-5-yl)-methoxycumarin

Ausbeute: 36 %; Fp. 205 °C (Methanol)
$C_{15}H_{13}BrN_2O_3S$ (381)
Ber. 47,26 C 3,44 H 20,96 Br 7,35 N 12,59 O 8,41 S Gef. 47,1 C 3,5 H 20,8 Br 7,4 N 12,7 O 8,5 S

Beispiel 68

7-(3-Cyclobutylisoxazol-5-yl)-methoxy-3,4-dimethylcumarin

Durchführung und Aufarbeitung erfolgten wie unter Beispiel 54 beschrieben.
Ausbeute:26 %; Fp. 100-101 °C (Methanol)
$C_{19}H_{19}NO_4$ (325)
Ber. 70,15 C 5,84 H 4,3 N 19,7 O
Gef. 69,9 C 5,9 H 4,6 N 19,5 O

Beispiel 69

7-(1-Benzyl-1,2,4-triazol-5-yl)-methoxy-3,4-dimethylcumarin

Ausbeute: 49 %; Fp. 151-152 °C (Methanol)
$C_{21}H_{19}N_3O_3$ (361)
Ber. 69,79 C 5,30 H 11,63 N 13,28 O
Gef. 69,3 C 5,5 H 11,8 N 13,6 O

Beispiel 70

7-(1-Isopropyl-1,2,4-triazol-5-yl)-methoxy-3,4-dimethylcumarin-hydrochlorid

Die Durchführung erfolgte wie unter Beispiel 1 beschrieben. Nach der Hydrolyse wurde mit Methylenchlorid extrahiert, die organische Phase eingeengt und der Rückstand säulenchromatographisch gereingt (Kieselgel; $CH_2Cl_2/CH_3OH$ 20:1). Die freie Base wurde in $CH_2Cl_2$ gelöst und mit etherischer HCl versetzt. Der gebildete Niederschlag wurde abgesaugt, mit wenig Ether gewaschen und getrocknet.
Ausbeute: 32 %; Fp. 179-181 °C
$C_{17}H_{20}ClN_3O_3$ (350)
Ber. 58,37 C 5,76 H 10,13 Cl 12,01 N 13,71 O
Gef. 57,9 C 5,9 H 10,0 Cl 12,1 N 14,2 O

Beispiel 71

3,4-Dimethyl-7-[1-(1,3-dimethyl-1,2,4-triazol-5-yl)]-ethoxycumarin

Ausbeute 32%; Fp. 160-162 °C (Methanol)
$C_{17}H_{19}N_3O_3$ (313)
Ber. 65,16 C 6,11 H 13,41 N 15,32 O
Gef. 65,1 C 6,2 H 13,3 N 15,6 O

Beispiel 72

7-(3-Isopropyl-1-methylpyrazol-5-yl)-methoxy-3,4-dimethylcumarin

Ausbeute: 37 %; Fp. 125-126 °C (Methanol)
$C_{19}H_{22}N_2O_3$ (326)
Ber. 69,92 C 6,79 H 8,58 N 14,71 O
Gef. 69,3 C 7,1 H 9,1 N 14,3 O

Beispiel 73

3,4-Dimethyl-7-(1,5-dimethylpyrazol-3-yl)-methoxycumarin

Ausbeute: 23 %; Fp. 169-171 °C (Methanol)
$C_{17}H_{18}N_2O_3$ (298)
Ber. 68,44 C 6,08 H 9,39 N 16,09 O
Gef. 68,2 C 6,3 H 9,1 N 16,3 O

Beispiel 74

7-(2-Benzylthiazol-4-yl)-methoxy-3,4-dimethylcumarin

Ausbeute: 21 %; Fp. 142-143 °C (Methanol)
$C_{22}H_9NO_3S$ (377)
Ber. 0,01 C 5,07 H 3,71 N 12,72 O 8,49 S
Gef. 69,6 C 5,2 H 3,7 N 13,0 O 8,4 S

Beispiel 75

7-(2-Isopropylthiazol-4-yl)-methoxy-3,4-dimethylcumarin

Ausbeute: 20 %; Fp. 103 °C (Methanol)
$C_{18}H_{19}NO_3S$ (329)
Ber. 65,63 C 5,81 H 4,25 N 14,57 O 9,79 S
Gef. 65,0 C 6,0 H 3,9 N 15,2 O 9,2 S

Beispiel 76

3,4,8-Trimethyl-7-(2-methyl-1,3,4-thiadiazol-5-yl)-methoxycumarin

Ausbeute: 62 %; Fp. 231-233 °C (Methanol)
$C_{16}H_{16}N_2O_3S$ (316)
Ber. 60,74 C 5,10 H 8,85 N 15,17 O 10,13 S
Gef. 60,5 C 5,2 H 9,0 N 15,1 O 10,3 S

Beispiel 77

3,4,5-Trimethyl-7-(2-methyl-1,3,4-thiadiazol-5-yl)-methoxycumarin

Ausbeute: 76 %; Fp. 201-203 °C (Methanol)
$C_{16}H_{16}N_2O_3S$ (316)
Ber. 60,74 C 5,10 H 8,85 N 15,17 O 10,13 S
Gef. 60,5 C 5,2 H 9,0 N 15,1 O 10,1 S

Beispiel 78

7-(3-Cyclopentylisoxazol-5-yl)-methoxy-3,4-dimethylcumarin

Die Durchführung erfolgte wie unter Beispiel 1 beschrieben. Nach der Hydrolyse wurde der ausgefallene Feststoff abgesaugt und 5 mal mit je 100 ml n-Heptan ausgekocht. Die vereinigten Heptanphasen wurden eingeengt und der Rückstand aus Methanol umkristallisiert.
Ausbeute: 15 %; Fp. 81-83 °C
$C_{20}H_{21}NO_4$ (339)
Ber. 70,78 C 6,24 H 4,13 N 18,86 O
Gef. 70,6 C 6,2 H 4,0 N 18,8 O

Beispiel 79

7-[3-(1-Methoxyethyl)-isoxazol-5-yl]-methoxy-3,4-dimethylcumarin

Die Reaktionsmischung wurde 12 h bei 70 °C gerührt. Ansatz und Aufarbeitung erfolgten wie unter Beispiel 1 beschrieben.
Ausbeute: 37 %; Fp. 124-127 °C (Methanol)
$C_{18}H_{19}NO_5$ (329)
Ber. 65,64 C 5,81 H 4,25 N 24,29 O
Gef. 65,4 C 6,0 H 4,2 N 24,7 O

Beispiel 80

7-(2-Cyclopropylthiazol-4-yl)-methoxy-3,4-dimethylcumarin

Die Reaktionsmischung rührte 12 h bei 70 °C. Ansatz und Aufarbeitung erfolgten wie unter Beispiel 1 beschrieben.
Ausbeute: 51 %; Fp. 151 °C (Methanol)
$C_{18}H_{17}NO_3S$ (327)
Ber. 66,03 C 5,23 H 4,28 N 14,66 O 9,79 S
Gef. 65,7 C 5,4 H 4,4 N 15,0 O 9,7 S

Beispiel 81

3,4-Dimethyl-7-(1-methylpyrazol-3-yl)-methoxycumarin

Ausbeute: 47 %; Fp.135 °C (Methanol)
$C_{16}H_{16}N_2O_3$ (284)
Ber. 67,59 C 5,67 H 9,85 N 16,88 O
Gef. 67,5 C 5,8 H 9,8 N 17,0 O

Beispiel 82

7-(5-Isopropyl-1-methylpyrazol-3-yl)-methoxy-3,4,5-trimethylcumarin

Ausbeute: 65 %, Fp. 128 °C (Methanol)
$C_{20}H_{24}N_2O_3$ (340)
Ber. 70,57 C 7,11 H 8,23 N 14,10 O
Gef. 70,3 C 7,1 H 8,1 N 14,5 O

Beispiel 83

7-(5-Isopropyl-1-methylpyrazol-3-yl)-methoxy-3,4,8-trimethylcumarin

Ausbeute: 46 %; Fp. 146-148 °C (Methanol)
$C_{20}H_{24}N_2O_3$ (340)
Ber. 70,57 C 7,11 H 8,23 N 14,10 O
Gef. 70,3 C 7,1 H 8,1 N 14,5 O

Beispiel 84

6-Ethyl-7-(5-isopropyl-1-methylpyrazol-3-yl)-methoxy-3,4-dimethylcumarin

Ausbeute: 51 %; Fp. 136-137 °C (Methanol)
$C_{21}H_{26}N_2O_3$ (354)
Ber. 71,16 C 7,39 H 7,90 N 13,54 O
Gef. 71,1 C 7,5 H 7,9 N 13,6 O

Beispiel 85

6-Ethyl-3,4-dimethyl-7-(2-methyl-1,3,4-thiadiazol-5-yl)-methoxycumarin

Ausbeute: 80 %; Fp. 201-203 °C (Methanol)
$C_{17}H_{18}N_2O_3S$ (330)
Ber. 61,80 C 5,49 H 8,48 N 14,53 O 9,70 S
Gef. 61,7 C 5,6 H 8,4 N 14,6 O 9,7 S

Beispiel 86

3,4-Dimethyl-7-[3-(pyridin-2-yl)-isoxazol-5-yl]-methoxycumarin

Ausbeute: 86 %; Fp. 193 ° C (Methanol)
$C_{20}H_{16}N_2O_4$ (348)
Ber. 68,96 C 4,63 H 8,04 N 18,37 O
Gef. 68,4 C 4,6 H 8,1 N 17,9 O

Beispiel 87

7-(3-Cyclohexylisoxazol-5-yl)-methoxy-3,4-dimethylcumarin

Die Durchführung und Aufarbeitung erfolgte wie unter Beispiel 78 beschrieben.
Ausbeute: 66 %; Fp. 122 ° C (n-Heptan)
$C_{21}H_{23}NO_4$ (353)
Ber. 71,73 C 6,56 H 3,86 N 18,11 O
Gef. 71,5 C 6,7 H 4,0 N 17,8 O

Beispiel 88

7-(3-t-Butyl-1-methylpyrazol-5-yl)-methoxy-3,4-dimethylcumarin

Ausbeute: 28 %; Fp. 143 ° C (Methanol)
$C_{20}H_{24}N_2O_3$ (340)
Ber. 70,57 C 7,11 H 8,23 N 14,10 O
Gef. 70,4 C 7,3 H 8,1 N 13,8 O

Beispiel 89

3,4-Dimethyl-7-(1,3-dimethyl-1,2,4-triazol-5-yl)-methoxycumarin

Ausbeute: 42 %; Fp. 180 ° C (Methanol)
$C_{16}H_{17}N_3O_3$ (299)
Ber. 64,20 C 5,72 H 14,04 N 16,04 O
Gef. 64,5 C 5,9 H 13,8 N 15,8 O

Beispiel 90

3,4-Dimethyl-7-(1-methyl-5-nitroimidazol-2-yl)-methoxycumarin

Ausbeute: 22 %; Fp. 184 ° C (Aceton)
$C_{16}H_{15}N_3O_5$ (329)
Ber. 58,36 C 4,59 H 12,76 N 24,29 O
Gef. 58,0 C 4,7 H 12,6 N 24,5 O

Beispiel 91

6-Brom-3,4-dimethyl-7-(1-methyl-5-isopropylpyrazol-3-yl)-methoxycumarin

Ausbeute: 32 %; Fp. 177 - 179 ° C (Methanol)

Beispiel 92

7-(2-t-Butylthiophen-5-yl)-methoxy-3,4-dimethylcumarin

Ausbeute: 86 %; Fp. 137 ° C (Essigsäureethylester)
$C_{20}H_{22}O_3S$ (342)

Ber. 70,15 C 6,48 H 14,02 O 9,36 S
Gef. 69,7 C 6,4 H 14,2 O 9,2 S

Beispiel 93

6-Brom-7-(2-t-butylthiophen-5-yl)-methoxy-3,4-dimethylcumarin

Ausbeute: 4,5 %; Fp. 170 °C (Methanol/Essigsäure)
$C_{20}H_{21}BrO_3S$ (421)
Ber. 50,01 C 5,02 H 18,96 Br 11,39 O 7,61 S
Gef. 56,8 C 5,0 H 18,3 Br 11,5 O 7,7 S

Beispiel 94

3,4-Dimethyl-7-(2-cyclopropyl-1,3,4-thiadiazol-5-yl)-methoxycumarin

Ausbeute: 62 %; Fp. 143 °C (Essigsäureethylester)
$C_{17}H_{16}N_2O_3S$ (328)
Ber. 62,18 C 4,91 H 8,53 N 14,62 O 9,86 S
Gef. 61,7 C 4,9 H 8,5 N 14,6 O 10,0 S

Beispiel 95

3,4-Dimethyl-7-(3,5-dimethylisoxazol-4-yl)-methoxycumarin

Ausbeute: 53 %; Fp. 145 - 147 °C (Methanol)
$C_{17}H_{17}NO_4$ (299)
Ber. 68,22 C 5,72 H 4,68 N 21,38 O
Gef. 67,9 C 5,9 H 4,5 N 21,8 O

Beispiel 96

3,4-Dimethyl-7-[3-(1-methylcyclopropyl)-isoxazol-5-yl]-methoxycumarin

Ausbeute: 64 %; Fp. 122 °C (Methanol)
$C_{19}H_{19}NO_4$ (325)
Ber. 70,14 C 5,89 H 4,31 N 19,69 O
Gef. 69,8 C 5,9 H 4,3 N 19,8 O

Beispiel 97

3,4-Dimethyl-7-[3-(tetrahydrofuran-3-yl)-isoxazol-5-yl]-methoxycumarin

Ausbeute: 48 %; Fp. 122 °C (Methanol)
$C_{20}H_{19}NO_5$ (325)
Ber. 66,85 C 5,61 H 4,1 N 23,43 O
Gef. 66,8 C 5,7 H 4,1 N 23,7 O

Beispiel 98

3,4-Dimethyl-7-(3-cyclopentylisoxazol-5-yl)-methoxycumarin

Ausbeute: 56 %; Fp. 128 °C (Methanol)
$C_{19}H_{20}N_2O_4$ (340)
Ber. 67,05 C 5,92 H 8,23 N 18,89 O
Gef. 66,9 C 6,0 H 8,1 N 18,9 O

Beispiel 99

3,4-Dimethyl-7-(3-cyclohexylisoxazol-5-yl)-methoxycumarin

Ausbeute: 77 %; Fp. 143 °C
$C_{20}H_{220}N_2O_4$ (354)
Ber. 67,78 C 6,26 H 7,90 N 18,06 O
Gef. 67,5 C 6,3 H 7,9 N 18,3 O

Beispiel 100

6-Chlor-3,4-dimethyl-7-(2-pyridinyl)-methoxycumarin

Durchführung und Aufarbeitung erfolgten wie unter Beispiel 2 beschrieben. Zur Reaktionsmischung wurden zusätzlich 0,1 % 18-Krone-6 zugefügt.
Ausbeute: 74 %; Fp. 201 °C (Methanol)
$C_{17}$-$H_{14}ClNO_3$ (316)
Ber. 64,67 C 4,47 H 11,23 Cl 4,44 N 15,20 O
Gef. 64,4 C 4,6 H 11,6 Cl 4,4 N 15,1 O

Beispiel 101

3,6-Dichlor-4-methyl-7-(5-methyl-1,3,4-thiadiazol-2-yl)-methoxycumarin

Ausbeute: 19,5 %; Fp. 216 °C (Methanol)
$C_{14}H_{10}Cl_2N_2O_3S$ (357)
Ber. 47,07 C 2,82 H 19,85 Cl 7,84 N 13,44 O 8,98 S
Gef. 46,9 C 2,9H 19,4 Cl 7,8 N 13,7 O 8,9 S

Beispiel 102

6-Brom-3-chlor-4-methyl-7-(1-methyl-5-isopropylpyrazol-3-yl)methoxycumarin

Durchführung und Aufarbeitung erfolgten analog Beispiel 100.
Ausbeute 46 %; Fp. 165 °C (Methanol)
$C_{18}H_{18}BrClN_2O_3$ (426)
Ber. 50,79 C 4,26 H 18,77 Br 8,33 Cl 6,58 N 11,27 O
Gef. 50,2 C 4,1 H 18,4 Br 8,7 Cl 6,4 N 11,5 O

Beispiel 103

3,6-Dichlor-4-methyl-7-(2-isopropylthiazol-4-yl)-methoxycumarin

Durchführung und Aufarbeitung erfolgten analog Beispiel 100.
Ausbeute: 28 %; Fp. 175 °C (Methanol)
$C_{17}H_{16}ClNO_3S$ (385)
Ber. 53,00 C 4,19 H 18,40 Cl 3,64 N 12,40 O 8,32 S
Gef. 52,8 C 4,0 H 18,5 Cl 3,6 N 12,5 O 8,3 S

Beispiel 104

6-Brom-3-chlor-4-methyl-7-(2-isopropylthiazol-4-yl)-methoxycumarin

Durchführung und Aufarbeitung erfolgten analog Beispiel 100.
Ausbeute: 22 %; Fp. 186 °C (Methanol)
$C_{17}H_{16}BrClNO_3S$ (428)
Ber. 47,60 C 3,50 H 18,60 Br 8,28 Cl 3,26 N 11,20 O 7,46 S
Gef. 47,7 C 3,6 H 18,7 Br 8,4 Cl 3,2 N 11,30 O 7,5 S

Beispiel 105

3,4-Dimethyl-7-(3-phenylisoxazol-5-yl)-methoxycumarin

Ausbeute: 73 %; Fp. 186 °C (Ethanol)
$C_{21}H_{17}NO_4$ (347)
Ber. 72,61 C 4,93 H 4,03 N 18,42 O
Gef. 72,6 C 5,1 H 3,9 N 18,4 O

Beispiel 106

3,4-Dimethyl-7-(4-methyloxazol-5-yl)-methoxycumarin Durchführung und Aufarbeitung erfolgten analog Beispiel 10.
Ausbeute: 34 %; Fp. 187 °C (Methanol)

Beispiel 107

4-Trifluormethyl-7-(2-methyl-1,3,4-thiadiazol-5-yl)-methoxycumarin

Durchführung und Aufarbeitung erfolgten analog Beispiel 10.
Ausbeute: 76 %, Fp. 178 °C (Methanol).
A) Auf einer Tablettenpresse werden in üblicher Weise Tabletten folgender Zusammensetzung gepreßt:
40,00 mg Substanz des Beispiels 108
120,00 mg Maisstärke
13,50 mg Gelatine
45,00 mg Milchzucker
2,25 mg Aerosil® (chemisch reine Kieselsäure in submikroskopisch feiner Verteilung)
6,75 mg Kartoffelstärke (als 6 %iger Kleister)
B) 20,00 mg Substanz des Beispiels 75
60,00 mg Kernmasse
Die Kernmasse besteht aus 9 Teilen Maisstärke, 3 Teilen Milchzucker und 1 Teil Luviskol® VA 64 (Vinylpyrrolidon-Vinylacetat-Mischpolymerisat 60 : 40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

## Patentansprüche

**1.** Heterocyclisch substituierte Alkoxycumarine der Formel

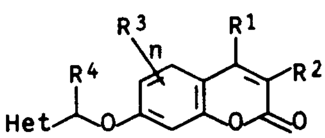

I,

in der
$R^1$ und $R^2$ unabhängig voneinander Wasserstoff, $C_1$- bis $C_5$-Alkyl, Trifluormethyl, Phenyl, Halogen oder gemeinsam eine $C_3$- bis $C_5$-Alkylenkette darstellen; ferner
$R^3$ $C_1$- bis $C_5$-Alkyl oder Halogen;
n eine ganze Zahl von 0 bis 3;
$R^4$ Wasserstoff oder $C_1$- bis $C_4$-Alkyl und
Het einen der folgenden Heterocyclen darstellt:

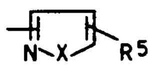 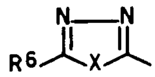 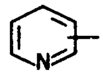 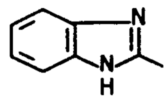 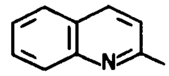 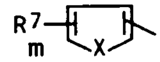

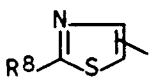 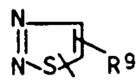 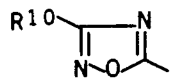 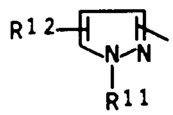 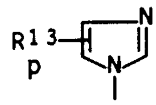 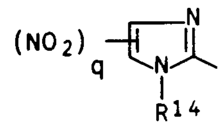

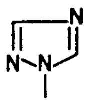 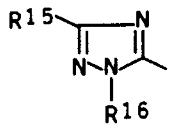 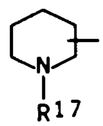 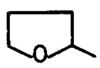 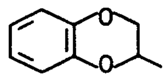 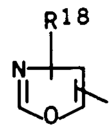 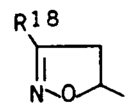

mit folgenden Bedeutungen für die benutzten Symbole:
X Sauerstoff oder Schwefel;
$R^5$
gegebenenfalls $C_1$-$C_4$-alkoxysubstituiertes $C_1$-$C_{10}$-Alkyl, 5-6-gliedriges Oxacycloalkyl, $C_3$-$C_7$-Cycloalkyl, Benzyl, einen gegebenenfalls durch Halogen oder $NO_2$ substituierten Phenylrest, einen Pyridinring, $C_1$-$C_5$-Alkoxycarbonyl, Perfluor$C_1$-$C_2$-alkyl;
$R^6$
$C_1$-$C_5$-Alkyl, $C_1$-$C_5$-Alkoxy;
$R^7$
$C_1$-$C_5$-Alkyl, einen gegebenenfalls halogensubstituierten Benzylrest, Halogen; m 0 bis 2;
$R^8$
$C_1$-$C_5$-Alkyl, $C_3$-$C_6$-Cycloalkyl, Benzyl, Phenyl;
$R^9$
Wasserstoff, $C_1$-$C_5$-Alkyl;
$R^{10}$
$C_1$-$C_5$-Alkyl, $C_1$-$C_6$-Cycloalkyl;
$R^{11}$
$C_1$-$C_5$-Alkyl;
$R^{12}$
Wasserstoff, $C_1$-$C_5$-Alkyl, $C_3$-$C_6$-Cycloalkyl;
$R^{13}$
$C_1$-$C_5$-Alkyl, Halogen, p = 0 bis 2;
$R^{14}$
$C_1$-$C_5$-Alkyl, q = 0 oder 1;
$R^{15}$, $R^{16}$
Wasserstoff, $C_1$-$C_5$-Alkyl, Benzyl;
$R^{17}$
Wasserstoff, $C_1$-$C_5$-Alkyl;
$R^{18}$
$C_1$-$C_5$-Alkyl
mit der Maßgabe, daß $R^5$ nicht Phenyl ist, wenn X Sauerstoff, $R^1$ Methyl und $R^2$ bis $R^4$ Wasserstoff darstellen.

2. Verfahren zur Herstellung der Verbindungen der Formel I nach Anspruch 1, dadurch gekennzeichnet, daß man ein Hydroxycumarin der Formel II oder deren Alkalimetallsalz mit einer Verbindung der Formel III

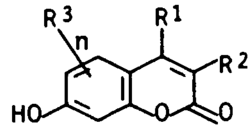 II, 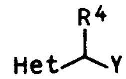 III,

wobei $R^1$ bis $R^4$, n und Het die im Anspruch 1 angegebenen Bedeutungen besitzen und Y für eine nucleofuge Abgangsgruppe steht, nach an sich bekannten Methoden umsetzt und isoliert.

3. Orales therapeutisches Mittel, das als Wirkstoff pro Dosis 10 bis 500 mg einer Verbindung der Formel I nach Anspruch 1, einschließlich der vom Anspruch 1 ausgenommenen Bedeutungskombinationen von $R^1$ bis $R^5$ und X, neben üblichen galenischen Hilfsmitteln enthält.

4. Parenterales therapeutisches Mittel, das als Wirkstoff pro Dosis 1 bis 50 mg einer Verbindung der Formel I nach Anspruch 1, einschließlich der vom Anspruch 1 ausgenommenen Bedeutungskombinationen von $R^1$ bis $R^5$ und X, neben üblichen galenischen Hilfsmitteln enthält.

5. Mittel zur Behandlung von Erkrankungen des zentralen Nervensystems, das als Wirkstoff eine Verbindung der Formel I nach Anspruch 1, einschließlich der vom Anspruch 1 ausgenommenen Bedeutungskombinationen von $R^1$ bis $R^5$ und X, neben üblichen galenischen Hilfsmitteln enthält.

## Claims

1. An alkoxycoumarin substituted by a heterocyclic radical, of the formula

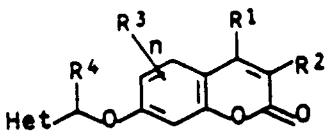

I

in which
$R^1$ and $R^2$ independently of one another are each hydrogen, $C_1$-$C_5$-alkyl, trifluoromethyl, phenyl or halogen, or together form a $C_3$-$C_5$-alkylene chain, $R^3$ is $C_1$-$C_5$-alkyl or halogen, n is an integer of from 0 to 3, $R^4$ is hydrogen or $C_1$-$C_4$-alkyl and Het is one of the following heterocyclic radicals:

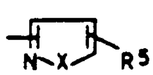 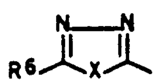 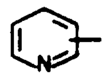 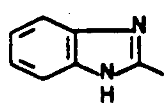 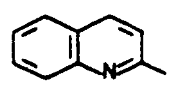 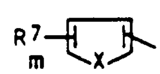

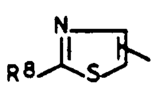 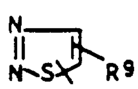 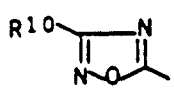 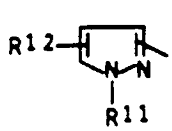 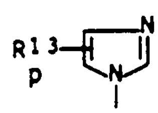 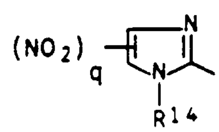

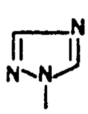 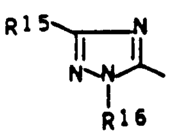 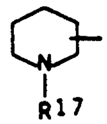 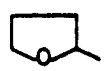 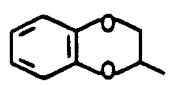 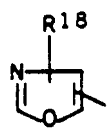 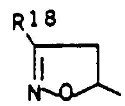

where X is oxygen or sulfur, $R^5$ is unsubstituted or $C_1$-$C_4$-alkoxy-substituted $C_1$-$C_{10}$-alkyl, 5-membered or 6-membered oxacycloalkyl, $C_3$-$C_7$-cycloalkyl, benzyl, phenyl which is unsubstituted or substituted by halogen or $NO_2$, a pyridine ring, $C_1$-$C_5$-alkoxycarbonyl or perfluoro-$C_1$-$C_2$-alkyl, $R^6$ is $C_1$-$C_5$-alkyl or $C_1$-$C_5$-alkoxy, $R^7$ is $C_1$-$C_5$-alkyl, unsubstituted or halogen-substituted benzyl or halogen, m is from 0 to 2, $R^8$ is $C_1$-$C_5$-alkyl, $C_3$-$C_6$-cycloalkyl, benzyl or phenyl, $R^9$ is hydrogen or $C_1$-$C_5$-alkyl, $R^{10}$ is $C_1$-$C_5$-alkyl or $C_1$-$C_6$-cycloalkyl, $R^{11}$ is $C_1$-$C_5$-alkyl, $R^{12}$ is hydrogen, $C_1$-$C_5$-alkyl or $C_3$-$C_6$-cycloalkyl, $R^{13}$ is $C_1$-$C_5$-alkyl or halogen, p is from 0 to 2, $R^{14}$ is $C_1$-$C_5$-alkyl, q is 0 or 1, $R^{15}$ and $R^{16}$ are each hydrogen, $C_1$-$C_5$-alkyl or benzyl, $R^{17}$ is hydrogen or $C_1$-$C_5$-alkyl and $R^{18}$ is $C_1$-$C_5$-alkyl, with the proviso that $R^5$ is not phenyl when X is oxygen, $R^1$ is methyl and $R^2$ to $R^4$ are each hydrogen.

2. A process for the preparation of a compound of the formula I as claimed in claim 1, wherein a hydroxycoumarin of the formula II or its alkali metal salt is reacted with a compound of the formula III

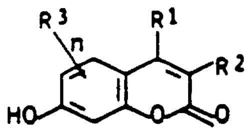 II, 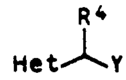 III,

where $R^1$ to $R^4$, n and Het have the meanings stated in claim 1 and Y is a nucleofugic leaving group, by a conventional method and the product is isolated.

**3.** An oral therapeutic agent which contains, as the active compound, from 10 to 500 mg, per dose, of a compound of the formula I as claimed in claim 1, including the combinations of meanings of $R^1$ to $R^5$ and X excluded from claim 1, in addition to conventional pharmaceutical auxiliaries.

**4.** A parenteral therapeutic agent which contains, as the active compound, from 1 to 50 mg, per dose, of a compound of the formula I as claimed in claim 1, including the combinations of meanings of $R^1$ to $R^5$ and X excluded from claim 1, in addition to conventional pharmaceutical auxiliaries.

**5.** An agent for treating disorders of the central nervous system, which contains, as the active compound, a compound of the formula I as claimed in claim 1, including the combinations of meanings of $R^1$ to $R^5$ and X excluded from claim 1, in addition to conventional pharmaceutical auxiliaries.

**Revendications**

**1.** Alcoxycoumarines à substitution hétérocyclique, répondant à la formule :

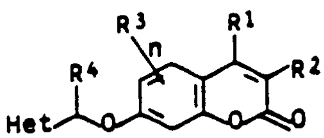 (I)

dans laquelle

$R^1$ et $R^2$ représentent, chacun indépendamment de l'autre, un atome d'hydrogène, un radical alkyle en $C_1$-$C_5$, trifluorométhyle, phényle, un atome d'halogène, ou bien ils représentent ensemble une chaîne alkylène en $C_3$-$C_5$ ; en outre,

$R^3$ représente un atome d'halogène ou un radical alkyle en $C_1$-$C_5$ ;

n représente un nombre entier qui varie de 0 à 3;

$R^4$ représente un atome d'hydrogène ou un radical alkyle en $C_1$-$C_4$, et

Het représente l'un des radicaux hétérocycliques qui suivent :

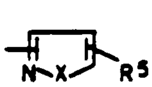 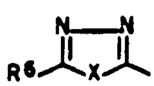 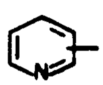 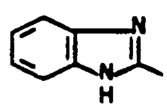 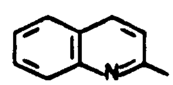 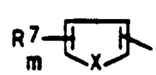

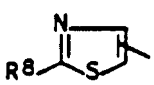 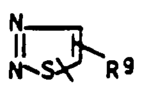 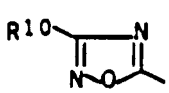 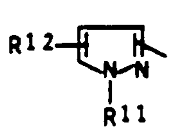 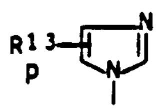 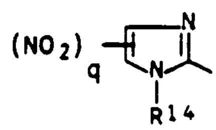

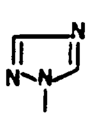 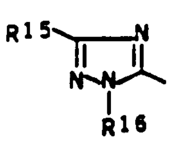 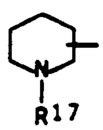 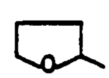 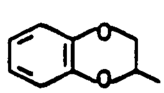 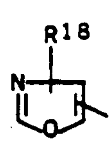 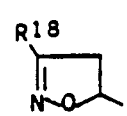

où les symboles utilisés ont les significations suivantes :

X représente l'oxygène ou le soufre;

$R^5$ représente un radical alkyle en $C_1$-$C_{10}$ à substitution alcoxylique en $C_1$-$C_4$ éventuelle, un radical oxacycloalkyle pentagonal ou hexagonal, un radical cycloalkyle en $C_3$-$C_7$, un radical benzyle, un radical phényle à substitution $NO_2$ ou halogénée éventuelle, un cycle pyridine, un radical alcoxy($C_1$-$C_5$)-carbonyle, un radical perfluoralkyle en $C_1$-$C_2$;

$R^6$ représente un radical alkyle en $C_1$-$C_5$, alcoxy en $C_1$-$C_5$;

$R^7$ représente un radical alkyle en $C_1$-$C_5$, un radical benzyle à substitution halogénée éventuelle, un atome d'halogène; m ayant une valeur qui varie de 0 à 2;

$R^8$ représente un radical alkyle en $C_1$-$C_5$,cycloalkyle en $C_3$-$C_6$, benzyle, phényle;

$R^9$ représente un atome d'hydrogène ou un radical alkyle en $C_1$-$C_5$;

$R^{10}$ représente un radical alkyle en $C_1$-$C_5$,cycloalkyle en $C_1$-$C_6$;

$R^{11}$ représente un radical alkyle en $C_1$-$C_5$;

$R^{12}$ représente un atome d'hydrogène, un radical alkyle en $C_1$-$C_5$, ou un radical cycloalkyle en $C_3$-$C_6$;

$R^{13}$ représente un radical alkyle en $C_1$-$C_5$, un atome d'halogène, p ayant une valeur qui varie de 0 à 2;

$R^{14}$ représente un radical alkyle en $C_1$-$C_5$, q étant égal à 0 ou à 1;

$R^{15}$, $R^{16}$ représentent des atomes d'hydrogène, des radicaux alkyle en $C_1$-$C_5$, benzyle;

$R^{17}$ représente un atome d'hydrogène ou un radical alkyle en $C_1$-$C_5$;

$R^{18}$ représente un radical alkyle en $C_1$-$C_5$,

avec la condition que $R^5$ ne représente pas de radical phényle lorsque X représente un atome d'oxygène, $R^1$ représente un radical méthyle et $R^2$ à $R^4$ représentent des atomes d'hydrogène.

**2.** Procédé de préparation des composés de la formule I suivant la revendication 1, caractérisé en ce que l'on fait réagir une hydroxycoumarine de la formule II ou un sel de métal alcalin de cette dernière avec un composé de la formule III :

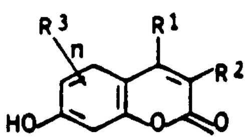 II, 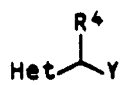 III,

dans lesquelles

$R^1$ à $R^4$, n et Het ont les significations qui leur ont été attribuées à propos de la définition de la revendication 1 et Y représente un groupe sortant nucléofuge, selon des procédés en soi connus et on isole le produit obtenu.

**3.** Composition thérapeutique orale, qui contient, à titre de principe actif par dose, 10 à 500 mg d'un composé de la formule I suivant la revendication 1, y compris les combinaisons de significations de $R^1$ à $R^5$ et X exclues de la revendication 1, outre des adjuvants ou additifs galéniques usuels.

**4.** Composition thérapeutique parentérale, qui contient, à titre de principe actif par dose, 1 à 50 mg d'un composé de la formule I suivant la revendication 1, y compris les combinaisons de significations de $R^1$ à $R^5$ et X exclues de la revendication 1, outre des adjuvants ou additifs galéniques usuels.

**5.** Composition pour le traitement de maladies du système nerveux central, qui contient, à titre de principe actif, un composé de la formule I selon la revendication 1, y compris les combinaisons de significations de $R^1$ à $R^5$ et X exclues de la revendication 1, outre des adjuvants ou additifs galéniques usuels.